(19)

Europäisches
Patentamt
European
Patent Office
Office européen
des brevets

(11) **EP 3 932 485 A1**

(12) **EUROPEAN PATENT APPLICATION**
published in accordance with Art. 153(4) EPC

(43) Date of publication:
**05.01.2022 Bulletin 2022/01**

(21) Application number: **20763421.3**

(22) Date of filing: **26.02.2020**

(51) Int Cl.:
*A61P 3/00* *(2006.01)*       *A61P 3/02* *(2006.01)*
*A61P 11/00* *(2006.01)*      *A61P 27/02* *(2006.01)*
*A61K 36/31* *(2006.01)*      *A61K 36/82* *(2006.01)*
*A61K 36/88* *(2006.01)*      *A61K 31/352* *(2006.01)*
*A61K 35/644* *(2015.01)*

(86) International application number:
**PCT/JP2020/007858**

(87) International publication number:
**WO 2020/175579 (03.09.2020 Gazette 2020/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.02.2019 JP 2019034822**

(71) Applicant: Otsuka Pharmaceutical Co., Ltd.
**Tokyo 101-8535 (JP)**

(72) Inventors:
• **IKEDA, Yasutaka**
  **Osaka-shi, Osaka 540-0021 (JP)**

• **MIZOKAMI, Tsubasa**
  **Osaka-shi, Osaka 540-0021 (JP)**
• **AKIYAMA, Minoru**
  **Osaka-shi, Osaka 540-0021 (JP)**
• **ABIRU, Yasuhiro**
  **Osaka-shi, Osaka 540-0021 (JP)**
• **OYAMA, Ayuko**
  **Osaka-shi, Osaka 540-0021 (JP)**

(74) Representative: **Hoffmann Eitle
Patent- und Rechtsanwälte PartmbB
Arabellastraße 30
81925 München (DE)**

(54) **COMPOSITION CONTAINING PLANT-DERIVED EXTRACT AND/OR PLANT-DERIVED PROCESSED PRODUCT**

(57)     The present invention provides a composition for improvement in physical activity efficiency, a composition for reducing fatigue, and a composition for improving dynamic/kinetic visual acuity, comprising a plant-derived extract and/or a plant-derived processed product.

**EP 3 932 485 A1**

Fig. 1

○ Placebo (n=20)   ● KMP 2.5 mg (n=19)   △ KMP 10 mg (n=20)   ✕ KMP 25 mg (n=19)

Single ingestion

Consecutive ingestion

Differences at all intensities were analyzed by two-way repeated measures ANOVA with Tukey-corrected post hoc tests.
KMP 2.5 mg: $^{a}P<0.01$, $^{a'}P<0.001$, $^{a''}P<0.0001$ vs Placebo; KMP 10 mg: $^{b}P<0.0001$ vs Placebo, $^{c}P<0.0001$ vs Placebo

**Description**

Technical Field

The present application relates to a composition for improvement in physical activity efficiency, a composition for reducing fatigue, and a composition for improving dynamic/kinetic visual acuity.

Background Art

Improvement of physical activity efficiency, reduction of fatigue, and improvement in dynamic/kinetic visual acuity are very important not only in athletes with intense training but also in ordinary people's daily work (e.g. housework, baggage handling, stair climbing). Oxygen consumption is an indicator of energy production, and improving oxygen consumption efficiency is key to achieving a sustained "physical activity" in sports and daily life without feeling tired or breathlessness.

Arterial oxygen saturation at rest is generally considered normal at 96% or higher, but decreases to 93-88% during strenuous exercise (Non-Patent Document 1). Arterial oxygen saturation (at rest) is about 97% in people in their 20s, but this value decreases with age, reaching about 93% in people in their 60s (Non-Patent Document 2). In other words, in addition to the rapid decline in oxygen status during intense sports, the decline in oxygen status may also occur in the daily lives of ordinary people due to aging, labor, bad weather (atmospheric depression), apnea syndrome and the like.

Since the decline in the oxygen state can occur not only in sports but also in the daily life of ordinary people, it is desired to provide a preparation which can improve the oxygen consumption efficiency, improve the physical activity efficiency, reduce the fatigue, or improve the dynamic/kinetic visual acuity, at the declined oxygen state in addition to at the normal oxygen state, and which can be taken safely on a daily basis.

Kaempferol is a kind of natural flavonoid contained in various edible plants, such as tea, broccoli, grapefruit, cabbage, kale, beans, *Cichorium endivia,* leek, tomato, strawberry, grape, Brussels sprouts, apple, quinua, horseradish, saffron, propolis, Takana, and arugula.

Natural flavonoids, including kaempferol, have been studied with a focus on their diverse physiological effects, including the involvement of kaempferol in mitochondrial function (Patent Document 1, Patent Document 2, and Non-Patent Document 3) and the effects of kaempferol on cellular energy expenditure and thyroid hormone (Non-Patent Document 4), all of which relate to in vitro studies.

Patent Document 3 discloses the effect of quercetin on lactic acid concentration, but there is no specific disclosure using other flavonoids.

Prior Art Document

Patent Document

Patent Document 1: WO 2014/171333
Patent Document 2: JP 2007-228855 A
Patent Document 3: JP 2013-542924 A

Non-Patent Document

Non-Patent Document 1: Williams JH, Powers SK, Stuart MK (1986) Hemoglobin desaturation in highly trained athletes during heavy exercise. Med Sci Sports Exerc 18: 168-173
Non-Patent Document 2: Wilkins, R.L. et al. Clinical assessment in respiratory care, (2004), Relationship between Age, PaO, and Saturation
Non-Patent Document 3: M. Montero, C.D. Lobaton, E. Hernandez-Sanmiguel, et al., Direct activation of the mitochondrial calcium uniporter by natural plant flavonoids, Biochem. J. 384 (2004) 19-24.
Non-Patent Document 4: da-Silva WS, Harney JW, Kim BW, Li J, Bianco SD, Crescenzi A, Christoffolete MA, Huang SA, Bianco AC 2007 The small polyphenolic molecule kaempferol increases cellular energy expenditure and thyroid hormone activation. Diabetes 56:767-776.
Non-Patent Document 5: ″Oxygen uptake effciency slope (OUES): Sono seirigaku-teki kiso to rinshō ōyo″ [Oxygen

uptake effciency slope (OUES): its physiological basis and clinical application], The Tokai journal of sports medical science 11, 9-14, 1999-03, Tokai University (in Japanese).

The disclosures of the prior art documents cited herein are hereby incorporated by reference in their entirety.

Summary

Technical Problem

An object of the present application is to provide a composition which can improve oxygen consumption efficiency (that is, the ability to use oxygen can be enhanced.), and thereby can suppress a decrease in physical activity efficiency, improve physical activity efficiency, or reduce fatigue, or which can suppress a decrease in dynamic/kinetic visual acuity, or improve dynamic/kinetic visual acuity, even at the lowered oxygen state in addition to the normal oxygen state.

Solution to Problem

The present inventors have been intensively studied to solve the above problems, and have found that oral administration of a kaempferol-containing composition to a human increases oxygen consumption efficiency, improves physical activity efficiency, reduces fatigue feeling, and improves dynamic/kinetic visual acuity, in a wide range of an exercise ranging from mild exercise of the level of daily work to an exercise of the level of intense sports. Furthermore, among edible plants known to contain kaempferol (including kaempferol glycosides), the present inventors have revealed that, especially, horseradish leaf, saffron, black tea leaf, kale, propolis, arugula, quinoa, and Takana contain a large amount of kaempferol or kaempferol glycosides, and have reached the present invention.

The present invention provides the following:

[1] A composition for improvement in physical activity efficiency, comprising a plant-derived extract selected from a horseradish extract, a saffron extract, a tea leaf extract, a kale extract, a propolis extract, a Takana extract, an arugula extract, and a mixture thereof, and/or a plant-derived processed product selected from a horseradish processed product, a saffron processed product, a tea leaf processed product, a kale processed product, a propolis processed product, a Takana processed product, an arugula processed product, and a mixture thereof.

[2] The composition according to [1], wherein the improvement in physical activity efficiency is improvement in endurance.

[3] The composition according to [1], wherein the improvement in physical activity efficiency is reduction of breathlessness.

[4] A composition for reducing fatigue, comprising a plant-derived extract selected from a horseradish extract, a saffron extract, a tea leaf extract, a kale extract, a propolis extract, a Takana extract, an arugula extract, and a mixture thereof, and/or a plant-derived processed product selected from a horseradish processed product, a saffron processed product, a tea leaf processed product, a kale processed product, a propolis processed product, a Takana processed product, an arugula processed product, and a mixture thereof.

[5] A composition for improving dynamic/kinetic visual acuity, comprising a plant-derived extract selected from a horseradish extract, a saffron extract, a tea leaf extract, a kale extract, a propolis extract, a Takana extract, an arugula extract, and a mixture thereof, and/or a plant-derived processed product selected from a horseradish processed product, a saffron processed product, a tea leaf processed product, a kale processed product, a propolis processed product, a Takana processed product, an arugula processed product, and a mixture thereof.

[6] The composition according to any one of [1] to [5], comprising a horseradish extract.

[7] The composition according to any one of [1] to [6], comprising a horseradish processed product.

[8] The composition according to any one of [1] to [7], wherein the plant-derived extract and/or the plant-derived processed product comprises kaempferol.

[9] The composition according to any one of [1] to [8], wherein the amount of kaempferol comprised in the composition is 0.1 mg to 200 mg.

[10] The composition according to any one of [1] to [9], wherein the amount of kaempferol comprised in the composition is 0.5 mg to 100 mg.

[11] The composition according to any one of [1] to [10], wherein said composition is for administration at a dose of 0.1 mg to 200 mg of kaempferol per administration.

[12] The composition according to any one of [1] to [11], wherein said composition is for administration at a dose of 0.5 mg to 100 mg of kaempferol per administration.

[13] The composition according to any one of [1] to [12], wherein said composition is for administration at a dose of 0.1 mg to 600 mg of kaempferol per day.

[14] The composition according to any one of [1] to [13], wherein said composition is for administration at a dose of 0.5 mg to 200 mg of kaempferol per day.

[15] The composition of any one of [1] to [14], wherein said composition is for administration to a subject who is hypoxic.

[16] The composition according to any one of [1] to [15], wherein said composition is a food and drink.

[17] The composition according to any one of [1] to [15], wherein said composition is a pharmaceutical composition.

Furthermore, the present invention provides use of a plant-derived extract selected from a horseradish extract, a saffron extract, a tea leaf extract, a kale extract, a propolis extract, a Takana extract, an arugula extract, and a mixture thereof, and/or a plant-derived processed product selected from a horseradish processed product, a saffron processed product, a tea leaf processed product, a kale processed product, a propolis processed product, a Takana processed product, an arugula processed product, and a mixture thereof, in the manufacture of a composition for improvement in physical activity efficiency, a composition for reducing fatigue, or a composition for improving dynamic/kinetic visual acuity.

Furthermore, the present invention provides a method for improvement in physical activity efficiency, a method for reducing fatigue, or a method for improving dynamic/kinetic visual acuity, comprising administering a plant-derived extract selected from a horseradish extract, a saffron extract, a tea leaf extract, a kale extract, a propolis extract, a Takana extract, an arugula extract, and a mixture thereof, and/or a plant-derived processed product selected from a horseradish processed product, a saffron processed product, a tea leaf processed product, a kale processed product, a propolis processed product, a Takana processed product, an arugula processed product, and a mixture thereof.

In addition, the present invention provides a plant-derived extract selected from a horseradish extract, a saffron extract, a tea leaf extract, a kale extract, a propolis extract, a Takana extract, an arugula extract, and a mixture thereof, or a plant-derived processed product selected from a horseradish processed product, a saffron processed product, a tea leaf processed product, a kale processed product, a propolis processed product, a Takana processed product, an arugula processed product, and a mixture thereof, for use in improvement in physical activity efficiency, reducing fatigue, or improving dynamic/kinetic visual acuity.

Effect of Invention

The composition of the present invention may improve oxygen consumption efficiency (an ability to utilize oxygen), thereby allowing for increased efficiency in any "physical activity" including daily activities and sports. For example, the composition of the present invention may allow for an exercise with a reduced breathlessness or an improved endurance. The composition of the present invention may also be used as a composition for reducing breathlessness or a composition for improving endurance. In addition, the composition of the present invention may reduce fatigue, and may allow for performing sports, daily housework, etc. without feeling fatigue. Furthermore, the composition of the present invention may improve dynamic/kinetic visual acuity and may contribute to an improved outcome, for example in sports.

Brief Description of Drawings

Fig.1 depicts a graph showing the oxygen consumption ($VO_2$) at each exercise intensity.

Fig.2 depicts a graph showing the oxygen consumption efficiency ($VO_2/VE$) after initiation of incremental loading exercise.

Fig.3 depicts a graph showing the oxygen uptake efficiency slope (OUES) for each of the dosages in incremental loading exercise.

Fig.4 depicts a graph showing the maximum oxygen consumption ($VO_{2peak}$) for each of the dosages in incremental loading exercise.

Fig.5 depicts a graph showing the maximum exercise load for each of the dosages in incremental loading exercise.

Fig.6-1 depicts a graph showing the exercise intensity (% HR) and the rating of perceived exertion (RPE) at the oxygen consumption equivalent to the level of climbing stairs.

Fig.6-2 depicts a graph showing the exercise intensity (% HR) and the rating of perceived exertion (RPE) at the oxygen consumption equivalent to the level of jogging.

Fig.7 depicts a graph showing the dynamic visual acuity (DVA) before and after incremental loading exercise.

Fig.8 depicts a graph showing the effect of each compound on ATP production in a hypoxic environment.

Fig.9-1 depicts a graph showing the ATP content in the soleus muscle (Sol).

Fig.9-2 depicts a graph showing the ATP content in the whole brain.

Fig.10 depicts a graph showing the amounts of kaempferol and kaempferol glycosides in the raw materials. KMP means kaempferol.

Description of Embodiments

The invention relates to a composition for improvement in physical activity efficiency, a composition for reducing fatigue, or a composition for improving dynamic/kinetic visual acuity, comprising a plant-derived extract selected from a horseradish extract, a saffron extract, a tea leaf extract, a kale extract, a propolis extract, a Takana extract, an arugula extract, and a mixture thereof (preferably a horseradish extract, more preferably a horseradish leaf extract), and/or a plant-derived processed product selected from a horseradish processed product, a saffron processed product, a tea leaf processed product, a kale processed product, a propolis processed product, a Takana processed product, an arugula processed product, and a mixture thereof(preferably a horseradish processed product, more preferably a horseradish leaf processed product).

In the present invention, a plant-derived extract is selected from a horseradish extract, a saffron extract, a tea leaf extract, a kale extract, a propolis extract, a Takana extract, and an arugula extract. In the present invention, one or more kinds of plant-derived extracts may be used in combination. In the present invention, "a mixture thereof" in the context of "a horseradish extract, a saffron extract, a tea leaf extract, a kale extract, a propolis extract, a Takana extract, an arugula extract, and a mixture thereof" means any combination of the listed extracts.

In the present invention, a plant-derived processed product is selected from a horseradish processed product, a saffron processed product, a tea leaf processed product, a kale processed product, a propolis processed product, a Takana processed product, and an arugula processed product. In the present invention, one or more kinds of plant-derived processed products may be used in combination. In the present invention, "a mixture thereof" in the context of a horseradish processed product, a saffron processed product, a tea leaf processed product, a kale processed product, a propolis processed product, a Takana processed product, an arugula processed product, and a mixture thereof" means any combination of the listed processed products.

In the present invention, a plant-derived extract means an extract obtained by subjecting a raw material (horseradish (for example, leaf thereof), saffron, tea leaf (for example, black tea leaf), kale, propolis, Takana, or arugula) to an enzymatic treatment and/or an acid hydrolysis treatment for converting kaempferol glycoside(s) into a kaempferol aglycone, and an extraction. In the enzymatic treatment, a microorganism capable of producing the enzyme may be used. The production of the plant-derived extract may include purification step(s) after the extraction.

In the present invention, a plant-derived processed product means a processed product obtained by subjecting a raw material (horseradish (for example, leaf thereof), saffron, tea leaf (for example, black tea leaf), kale, propolis, Takana, or arugula) to an enzymatic treatment and/or an acid hydrolysis treatment for converting kaempferol glycoside(s) into a kaempferol aglycone, without including an extraction. In the enzymatic treatment, a microorganism capable of producing the enzyme may be used.

In the present invention, the term "a horseradish extract" means a plant-derived extract obtained by using horseradish *(Armoracia rusticana)* as a raw material. In the present invention, the term "a horseradish processed product" means a plant-derived processed product obtained by using horseradish *(Armoracia rusticana)* as a raw material. Since leaf of horseradish contains a large amount of kaempferol glycoside(s) (for example, kaempferol 3-O-xylosylgalactoside), the leaf is preferably used as a raw material. In the present disclosure, the terms "a horseradish leaf extract" and "a horseradish leaf processed product" respectively mean a horseradish extract and a horseradish processed product which are produced by mainly using leaf of horseradish.

In the present invention, the term "a saffron extract" means a plant-derived extract obtained by using saffron *(Crocus sativus)* as a raw material. In the present invention, the term "a saffron processed product" means a plant-derived processed product obtained by using saffron *(Crocus sativus)* as a raw material. Saffron is known to contain kaempferol 7-O-β-d-glucopyranoside (Mini Rev Med Chem. 11(4):298-344.2011).

In the present invention, the term "a tea leaf extract" means a plant-derived extract obtained by using leaf of tea *(Camellia sinensis)* as a raw material. In the present invention, the term "a tea leaf processed product" means a plant-derived processed product obtained by using leaf of tea *(Camellia sinensis)* as a raw material. The tea leaf may be in a raw state or may a dried and/or fermented product (for example, green tea leaf, oolong tea leaf, black tea leaf). Tea leaf is known to contain kaempferol 3-O-(2-O-β-D-galactopyranosyl-6-O-α-L-rhamnopyranosyl)-β-D-glucopyranoside, kaempferol 3-O-(2-O-β-D-xylopyranosyl-6-O-α-L-rhamnopyranosyl)-β-D-glucopyranoside, kaempferol 3-glucosyl-(1→3)-rhamnosyl (1→6) galactoside, kaempferol 3-O-[2-coumaroyl-3-O-β-D-glucosyl-3-O-β-D-glucosylrutinoside] (Mini Rev Med Chem. 11(4):298-344.2011).

In the present invention, the term "a kale extract" means a plant-derived extract obtained by using kale *(Brassica oleracea)* as a raw material. In the present invention, a kale processed product means a plant-derived processed product obtained by using kale *(Brassica oleracea)* as a raw material. Kale is known to contain kaempferol 3-sinapoyl-di-glucoside-7-di-glucoside (Mini Rev Med Chem. 11(4):298-344.2011).

In the present invention, the term "a propolis extract" means a plant-derived extract obtained by using propolis as a raw material. In the present invention, the term "a propolis processed product" means a plant-derived processed product obtained by using propolis as a raw material.

In the present invention, the term "a Takana extract" means a plant-derived extract obtained by using Takana *(Brassica juncea)* as a raw material. In the present invention, the term "a Takana processed product" means a plant-derived processed product obtained by using Takana *(Brassica juncea)* as a raw material. Takana is known to contain kaempferol 7-O-β-D-glucopyranosyl-(1→3)-[β-D-glucopyranosyl- (1→6)]-glucopyranoside (Mini Rev Med Chem. 11(4) :298-344.2011) .

In the present invention, the term "an arugula extract" means a plant-derived extract obtained by using arugula *(Eruca sativa)* as a raw material. In the present invention, the term "an arugula processed product" means a plant-derived processed product obtained by using arugula *(Eruca sativa)* as a raw material. Arugula is known to contain kaempferol di-O-glycoside (Mini Rev Med Chem. 11(4) :298-344.2011) .

In producing a plant-derived extract/ a plant-derived processed product, a raw material may be in a raw state, or may be a dried product or a semidried product, From the viewpoint of efficiently performing an enzymatic treatment / an acid hydrolysis treatment / an extraction of kaempferol or its glycoside(s), it is preferable to use the raw material after pulverizing by a usually-used method.

A method of the extraction is not particularly limited, and the extraction may be performed by a method usually used in the field of manufacturing pharmacy or food engineering. For example, the extraction may be performed by using one or more organic solvents selected from the group consisting of ethanol, methanol, butanol, ether, ethyl acetate and chloroform, or a mixed solvent of the organic solvent(s) and water.

Plants usually contain more glycosides than their aglycones. However, a glycoside other than glucose glycoside is generally less easily absorbed in the body than its aglycone. In the present invention, the production process of a plant-derived extract and a plant-derived processed product comprises an enzymatic treatment / an acid hydrolysis treatment for converting kaempferol glycoside(s) to kaempferol aglycone. Examples of kaempferol glycoside that may be contained in the above-listed raw materials of a plant-derived extract include kaempferol glycosides wherein glucose, galactose, rhamnose, xylose, or a combination thereof (for example, sophorose (glucose + glucose), rutinose (rhamnose + glucose), neohesperidose (rhamnose + glucose)) is (are) O-glycosidically linked at the 3 position, the 6 position and/or the 7 position of kaempferol. Glycoside sugars that may be contained in the raw materials are exemplified below:

saffron: glucose;
tea leaf: glucose, galactose + rhamnose + glucose, xylose + rhamnose + glucose, galactose + glucose, glucose + rutinose (rhamnose + glucose);
kale: sophorose (glucose + glucose);
Takana: glucose + glucose + glucose;
arugula: glucose;
horseradish leaf: galactose + xylose.

The enzymatic treatment may be carried out using enzyme(s) optionally in combination suitable for converting glycoside(s) which may be contained in a raw material into the aglycone. Examples of the enzyme include glucosidase, arabinosidase, rhamnosidase, xylosidase, cellulase, hesperidinase, naringinase, glucuronidase, pectinase, galactosidase, amyloglucosidase, and amylase. The enzymatic treatment may be carried out using microorganism(s) capable of producing the enzyme(s), optionally in combination. Examples of the microorganism includes genus *Aspergillus,* genus *Bacillus,* genus *Penicillium,* genus *Rhizopus,* genus *Rhizomucor,* genus *Talaromyces,* genus *Bifidobacterium,* genus *Mortierella,* genus *Cryptococcus,* and genus *Microbacterium.*

For example, in a production process of a horseradish extract, the enzymatic treatment may be performed using glucosidase and xylanase (or microorganism(s) capable of producing them).

Alternatively, the glycoside(s) may be converted into the aglycone by an acid hydrolysis treatment.

In the present application, a high concentration of kaempferol aglycone in a plant-derived extract/a plant-derived processed product is preferable, but a plant-derived extract and a plant-derived processed product may be used, so long as they contain enough kaempferol aglycone for use as an ingredient for a food composition or a pharmaceutical composition (for example, 1 mg/g or more, or 3mg/g or more, preferably 10 mg/g or more, more preferably 20 mg/g or more, more preferably 50 mg/g or more). Kaempferol glycoside(s) may remain in a plant-derived extract/ a plant-derived processed product. A plant-derived extract and a plant-derived processed product may contain kaempferol 3-O-glucoside.

A method for quantifying kaempferol aglycone in a plant-derived extract and a plant-derived processed product is not particularly limited and may be performed by a usually-used method, but may be performed, for example, according to the method of Test Example 6 of this application.

In a production process of a plant-derived extract, the orders and numbers of an extraction and an enzymatic treatment / an acid hydrolysis treatment are not particularly limited. For example,

the production process may comprise steps where a raw material is subjected to an extraction with a solvent, and then the resulting extract is subjected to an enzymatic treatment / an acid hydrolysis treatment;

the production process may comprise steps where a raw material is subjected to an enzymatic treatment / an acid hydrolysis treatment, and then the resulting treated product is subjected to an extraction with a solvent; or

the production process may comprise steps where a raw material subjected to an extraction with a solvent, the resulting extract is subjected to an enzymatic treatment /an acid hydrolysis treatment, and the resulting treated product is subjected to an extraction with a solvent.

These exemplary production processes may further comprise step(s) where a resulting extract is subjected to a purification treatment and/or a concentration treatment.

In the present invention, a plant-derived extract/ a plant-derived processed product may be a product diluted with a diluent such as water, alcohol, sugar, etc. or may be a lyophilized powder.

For example, a horseradish extract is produced by the following steps (1) to (6).

(1) Horseradish leaves are harvested, washed, sterilized, and dried by hot air.
(2) The dried horseradish leaves are extracted with ethanol containing 0 to 50 v/v% water.
(3) The extracted liquid is purified through adsorption on and elution from a resin column.
(4) The purified liquid is adjusted to pH 3 -7 with an appropriate acid or base, and subjected to an enzymatic treatment with a hydrolase at 30 to 50 °C for an appropriate time.
(5) The enzyme is inactivated at 60 °C or higher.
(6) Lyophilization is performed to provide a horseradish extract (powder).

The compositions of the present invention may be such that a plant-derived extract and/or a plant-derived processed product are administered, for example, at 0.05 mg to 5 g per administration, preferably 0.1 mg to 3 g per administration, more preferably 0.5 mg to 2 g per administration, further more preferably 10 mg to 1.5 g per administration.

Examples of the lower limit of the dose of a plant-derived extract/a plant-derived processed product per administration include 0.05 mg, 0.1 mg, 0.5 mg, 10 mg, 20 mg, 50 mg, 100 mg, 200 mg, 500 mg, and 700 mg, and examples of the upper limit include 1 g, 1.5 g, 2 g, 3 g, and 5 g, and a preferred range of the dose of a plant-derived extract / a plant-derived processed product per administration can be indicated by a combination of the upper and lower limits.

In the compositions of the present invention, a plant-derived extract and/or a plant-derived processed product may be administered, for example, from 0.05 mg to 10 g per day, preferably from 0.1 mg to 7.5 g per day, more preferably from 0.5 mg to 5 g per day. Examples of the lower limit of the dose of a plant-derived extract / a plant-derived processed product per day include 0.05 mg, 0.1 mg, 0.5 mg, 10 mg, 20 mg, 50 mg, 200 mg, 500 mg, and 700 mg, and examples of the upper limit include 1 g, 1.5 g, 2 g, 3 g, 5 g, 7.5 g, and 10 g, and a preferred range of the dose of a plant-derived extract / a plant-derived processed product per day may be indicated by a combination of the upper and lower limits. The dose of a plant-derived extract and/or a plant-derived processed product which may be administered per day may be administered in a single dose or in multiple divided doses (for example, twice, three times, four times, and five times).

The amount of a plant-derived extract and/or a plant-derived processed product (or kaempferol) which may contain in the composition of the present invention (food and drink, pharmaceutical composition, etc.), the amount of a plant-derived extract and/or a plant-derived processed product (or kaempferol) administered per administration, and the amount of a plant-derived extract and/or a plant-derived processed product (or kaempferol) administered per day are not particularly limited as long as they are within the range in which the intended effect may be exerted, and may be selected according to the form of the composition, the number of administrations, the health condition of the subject, etc. The administration period of the composition of the present invention is not particularly limited as long as it is within the range in which the intended effect may be exerted, and may be administered as a single dose or continuously. In order to obtain a continuous effect of improvement in physical activity efficiency, reducing fatigue, or improving dynamic/kinetic visual acuity, the composition of the present invention may be desirably administered continuously over a long period of time, for example, 2 days, 3 days, 1 week, 10 days, 1 month, or 3 months or more.

Examples of the amount of a plant-derived extract and/or a plant-derived processed product comprised in the composition of the present invention, as the amount of kaempferol, includes 0.1 mg to 200 mg, preferably 0.5 mg to 100 mg, more preferably 1 mg to 30 mg, and more further preferably 2 mg to 10 mg, which may vary depending on the total weight of the composition. Examples of the lower limit of the amount of kaempferol include 0.1 mg, 0.5 mg, 1 mg, 2 mg, and 2.5 mg, and examples of the upper limit include 200 mg, 150 mg, 100 mg, 50 mg, 30 mg, 25 mg, 15 mg, 10 mg, 5 mg, 3 mg, and 2.5 mg, and a preferred range of the amount of kaempferol may be indicated by a combination of the upper and lower limits.

The compositions of the present invention may be such that a plant-derived extract and/or a plant-derived processed product are administered, for example, at 0.1 mg to 200 mg per administration, preferably 0.5 mg to 100 mg per administration, more preferably 1 mg to 30 mg per administration, and more further preferably 2 mg to 10 mg per administration, as kaempferol. Examples of the lower limit of the dose of kaempferol per administration include 0.1 mg, 0.5 mg, 1 mg, 2 mg, and 2.5 mg, and examples of the upper limit include 200 mg, 150 mg, 100 mg, 50 mg, 30 mg, 25 mg, 15 mg, 10 mg, 5 mg, 3 mg, and 2.5 mg, and a preferred range of the dose of kaempferol per administration can be indicated by a combination of the upper and lower limits.

In the compositions of the present invention, a plant-derived extract and/or a plant-derived processed product may be administered, for example, at 0.1 mg to 600 mg per day, preferably 0.5 mg to 200 mg per day, more preferably 1 mg to 100 mg per day, as kaempferol. Examples of the lower limit of the dose of kaempferol per day include 0.1 mg, 0.5 mg, 1 mg, 2 mg, and 2.5 mg, and examples of the upper limit include 600 mg, 300 mg, 200 mg, 150 mg, 100 mg, 50 mg, 30 mg, 25 mg, 15 mg, 10 mg, 5 mg, 3 mg, and 2.5 mg, and a preferred range of the dose of kaempferol per day may be indicated by a combination of the upper and lower limits. The dose of kaempferol which may be administered per day may be administered in a single dose or in multiple divided doses (for example, twice, three times, four times, and five times).

The composition of the present invention is preferably formulated as oral dosage forms, and the form is not particularly limited, but may be conventional food forms such as tablets, granules, capsules, powders, chewable tablets, sweets (cookies, biscuits, chocolate confectioneries, chips, cakes, gums, candies, gummies, buns, yokan (sweet bean jelly), puddings, jellies, yogurt, ice cream, sherbet, etc.), breads, noodles, rice, cereal foods, beverages (liquid preparations, soft drinks, carbonated drinks, nutritional drinks, powdered drinks, fruit drinks, milk drinks, jelly drinks, etc.), soups (powder, freeze-dry), miso soups (powder, freeze-dry), and the like.

The composition of the present invention may be a food and drink or a pharmaceutical composition, and may be used as a food and drink, for example Foods with Functional Claims, Food for specified health uses, a health food, a nutritional supplement (supplement), a food for medical use, etc.

The compositions of the invention may be formulated into orally administered formulations by adding pharmaceutically acceptable base(s), carrier(s), and/or additive(s) usable in foods, etc., in addition to a plant-derived extract / a plant-derived processed product. It is desirable that ingredients other than a plant-derived extract / a plant-derived processed product used in the composition of the present invention do not impair the stability of kaempferol, and that they do not impair the intended effect(s) of the composition of the present invention (for example, improved oxygen consumption, improved physical activity efficiency, reduced fatigue, or improved dynamic/kinetic visual acuity).

In the present invention, "improvement of oxygen consumption efficiency" means an increased ability to utilize oxygen. Specific examples include an increase in the oxygen consumption efficiency ($VO_2/VE$) described in the present Examples, and under a given exercise intensity an increase in oxygen consumption ($VO_2$), an increase in oxygen uptake efficiency slope (increase in OUES), and an increase in maximum oxygen consumption ($VO_{2peak}$).

As used herein, "physical activity" means a moving the body and includes any movements, for example daily housework, baggage handling, stair climbing, sports, and the like.

In the present invention, "improvement in physical activity efficiency" means that the body can be moved more easily in any kinds of physical activities. For example, improvement in physical activity efficiency includes continuing a physical activity for a long time in a more comfortable state by improving endurance, or doing a physical activity more easily in a state of reduced breathlessness. Examples of indices of improvement in physical activity efficiency include under a given exercise intensity, increased oxygen consumption ($VO_2$), increased oxygen consumption efficiency ($VO_2/VE$), increased oxygen uptake efficiency slope (increased OUES), increased maximum oxygen consumption ($VO_{2peak}$), increased maximum exercise load, decreased exercise intensity at a given oxygen consumption($VO_2$), or decrease in rating of perceived exertion (these terms are described in Examples).

When the composition of the present invention is administered to improve physical activity efficiency, the dosage and the number of doses are not particularly limited, and the composition may be administered in the dosage, the number of doses and the period of administration exemplified above.

In the present invention, the term "reducing fatigue" means that any physical activity can be done while suppressing fatigue. Examples of indices of fatigue reduction include decreased exercise intensity or decrease in rating of perceived exertion (these terms are described in Examples). When the composition of the present invention is administered for reducing fatigue, the dosage and the number of doses are not particularly limited, and may be administered, for example, at the dose, the number of doses and the period of administration exemplified above.

In the present invention, improvement of dynamic/kinetic visual acuity means prevention of reduction of dynamic/kinetic visual acuity or improvement of dynamic/kinetic visual acuity. When the composition of the present invention is administered for improving dynamic/kinetic visual acuity, the dosage and the number of doses are not particularly limited, and the composition may be administered in the dosage, the number of doses and the period of administration exemplified above.

The composition of the present invention may have an effect of improving oxygen consumption efficiency (That is, the ability to use oxygen increases.). Therefore, the composition of the present invention may also be used for improving oxygen consumption efficiency.

In the present invention, the "hypoxic" state means a state in which oxygen level in the body is insufficient, for example, a state in which arterial oxygen saturation is less than 95%. Since the composition of the present invention may have an effect of improving oxygen consumption efficiency even in a subject in a hypoxic state, the composition of the present invention may contribute to improvement of physical activity efficiency, reduction of fatigue, and improvement of dynamic/kinetic visual acuity even in the subject in the hypoxic state.

The subject of administration of the composition of the present invention is not particularly limited, but is preferably human. It is preferable to administer the composition before and after sports, before and after outdoor work, before and after daily work (going up and down stairs, doing housework, etc.), when the subject feels that daily fatigue cannot be relieved, when the subject wants to work efficiently, or when the subject feels that the physical movement has become dull due to aging.

Example

The present invention is explained in further detail with reference to Formulation Examples and Test Examples. However, the scope of the invention is not limited to these Examples.

[Formulation Example 1]

| Cookies (kaempferol content: 2.5 mg / piece) | |
| --- | --- |
| Quinua extract* | 37 wt% |
| Maple syrup | 22 wt% |
| Milk | 22 wt% |
| Salted butter | 15 wt% |
| Granulated sugar | 4 wt% |
| Total | 100 wt% |

According to a conventional method, these were mixed and baked at an oven temperature of about 140°C for 20 minutes to produce cookies. The amount of kaempferol contained in one cookie was 2.5 mg (HPLC).

Quinua extract*: an Quinua extract wherein kaempferol glycosides were degraded into kaempferol aglycone by an enzymatic treatment.

<Test Example 1: Incremental loading exercise test with bicycle >

For 25 healthy adult males, 3 dosages of kaempferol-containing cookie-shaped foods (containing 2.5 mg, 10 mg, or 25 mg of kaempferol, respectively) and a placebo cookie-shaped food (kaempferol-free) were used as test foods, and a once-a-day ingestion for consecutive 8 days were repeated four times by the crossover method. 3 hours after a test food ingestion on day 1 (Single ingestion) and on day 8 (Consecutive ingestion), an incremental loading exercise with bicycle was performed while sampling the exhaled gas to determine the oxygen consumption. The Heart rate and the rating of perceived exertion were monitored during exercise. Dynamic/kinetic visual acuity was measured before and after the exercise. Details of each endpoint are given below.

<1: Evaluation of oxygen consumption ($VO_2$) >

Oxygen consumption ($VO_2$) (mL/min/kg) was calculated from the difference between the amount of oxygen in the inhaled gas (atmosphere) and the amount of oxygen in the exhaled gas. When a weight of the pedal approaches a subject's limit during incremental loading exercise with bicycle, a heart rate (HR) is maximized. The increase in heart rate from the resting heart rate to the maximum heart rate was defined as 100% of exercise intensity, and the oxygen consumption ($VO_2$) was plotted at each exercise intensity.

That is, for example, an exercise intensity of 50% HR is calculated by the following equation:

```
100 × (x - Resting heart rate)/(Maximum heart rate -
Resting heart rate) = 50% HR, and the oxygen consumption
(VO2) in which the heart rate is the "x" means "oxygen
consumption (VO2) at an exercise intensity of 50% HR".
```

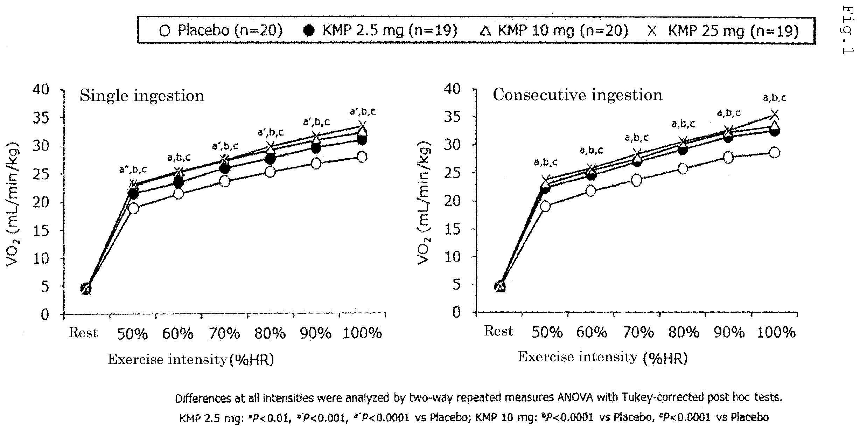

The results are shown in Fig. 1.

As shown in Fig. 1, in both of the single ingestion and the consecutive ingestion, compared with the no intake of kaempferol, the intake of kaempferol showed the increased oxygen consumption at all exercise intensities (50%, 60%, 70%, 80%, 90%, and 100%) .

<2: Evaluation of oxygen consumption efficiency (VO$_2$/VE) >

Oxygen consumption efficiency was calculated by the following equation.

$$\text{Oxygen consumption efficiency } (VO_2/VE) = \text{oxygen consumption / ventilation}$$

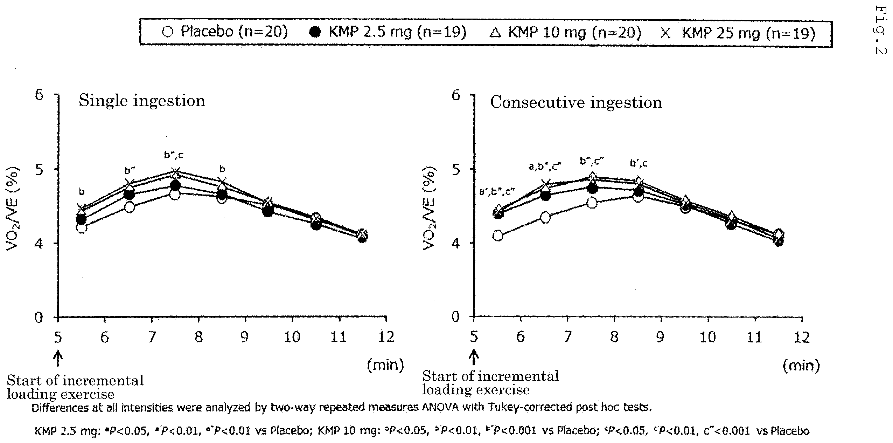

As shown in Fig. 2, in both of the single ingestion and the consecutive ingestion, compared with the no intake of kaempferol, the intake of kaempferol showed the increased oxygen consumption efficiency (VO$_2$/VE).

<3: Evaluation of oxygen uptake efficiency slope (OUES)>

The oxygen uptake efficiency slope (OUES) was calculated using the ventilation and VO$_2$ every 1 min from the beginning of the incremental loading exercise. Specifically, a graph of linearity was obtained by plotting "logarithmic value of ventilation (VE)" on the horizontal axis and "VO$_2$" on the vertical axis, and the slope of the linear function graph was defined as OUES. For details, see Non-Patent Document 5.

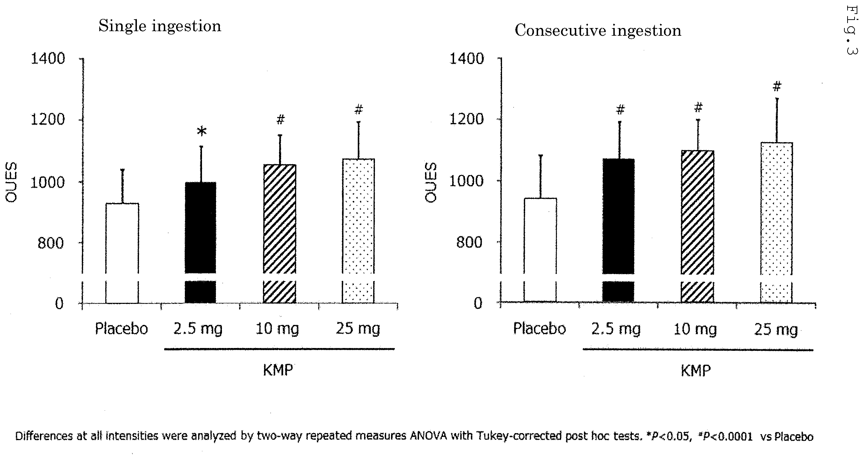

As shown in Fig. 3, in both of the single ingestion and consecutive ingestion, compared with the no intake of kaempferol, the intake of kaempferol showed the increased oxygen uptake efficiency slope (OUES), which indicates that oxygen was more efficiently utilized during incremental loading exercise.

<4: Evaluation of maximum oxygen consumption (VO$_{2peak}$) >

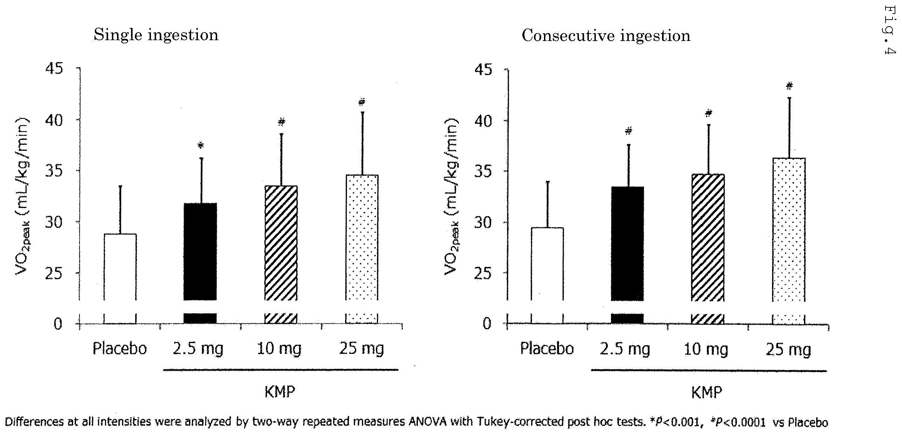

As shown in Fig. 4, in both of the single ingestion and consecutive ingestion, compared with the no intake of kaempferol, the intake of kaempferol showed the increased maximum oxygen consumption (VO$_{2peak}$) (mL/min/kg) .

<5: Evaluation of the maximum exercise load>

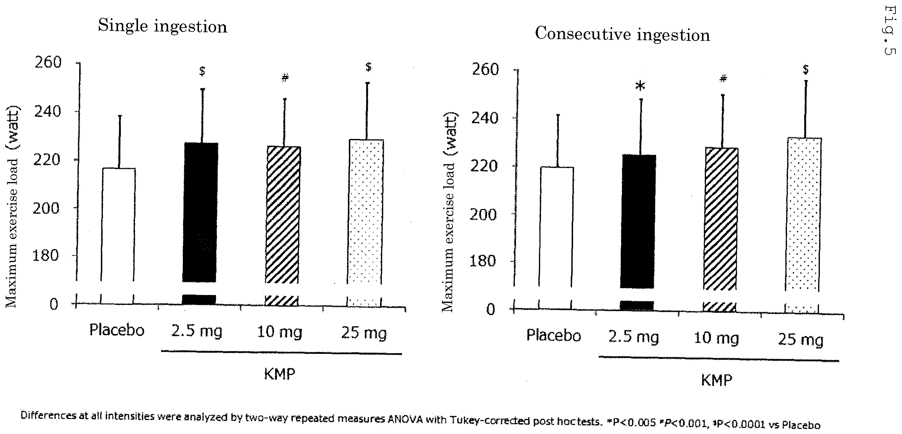

As shown in Fig. 5, in the both of the single ingestion and consecutive ingestion, compared with the no intake of kaempferol, the intake of kaempferol showed the increased maximum exercise load (Pedal Weight (watt)).

<6: Effects on exercise intensity of the level of daily work>

Oxygen consumption (VO$_2$) during stair climbing and jogging has been reported to be generally 14 mL/min/kg and 24.5 mL/min/kg, respectively.

Exercise intensity (% HR) and rating of perceived exertion (RPE) at the oxygen consumption (VO$_2$) of 14 mL/min/kg and 24.5 mL/min/kg in incremental loading exercise (the levels of stair climbing and jogging, respectively) were evaluated.

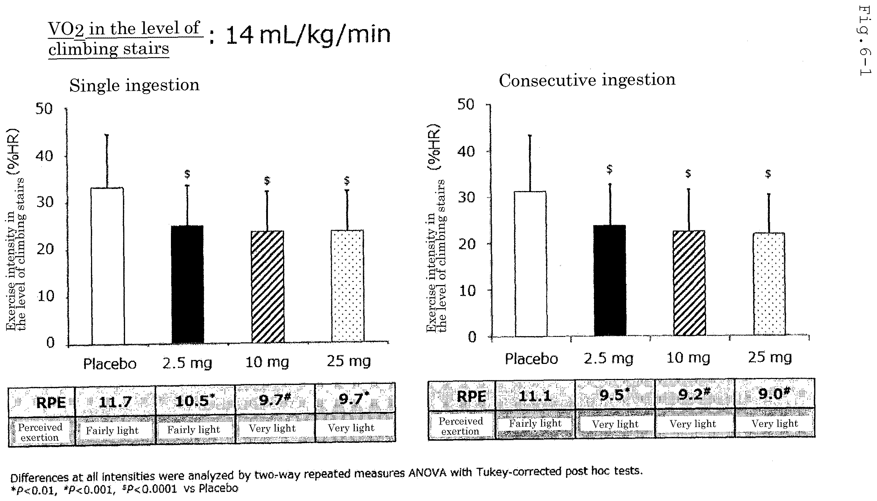

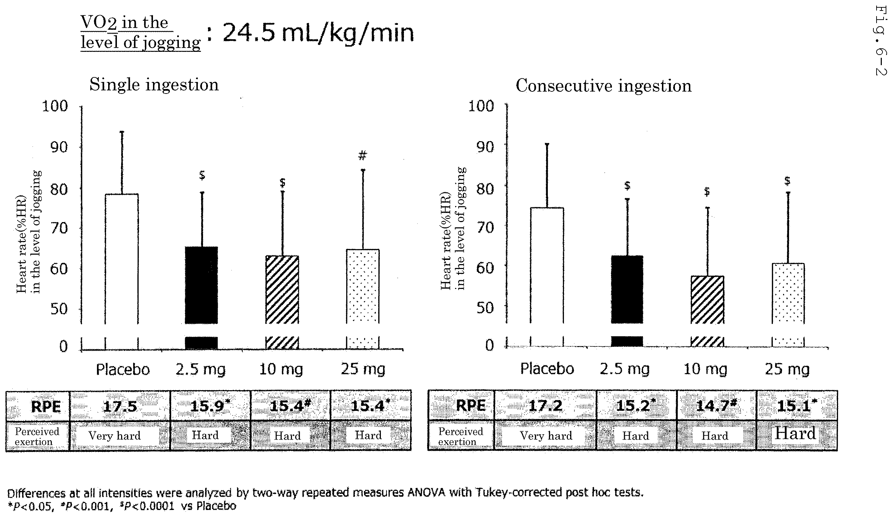

The calculation methods for oxygen consumption (VO$_2$) and exercise intensity (% HR) are the same as those described above. The perceived exertion was rated by the subject according to the following table at 1-minute intervals from the beginning of the incremental loading exercise, and the scales rated by each subject were averaged to give the rating of perceived exertion (RPE). As shown in Figs. 6-1 and 6-2, in the both of the single ingestion and consecutive ingestion, compared with the no intake of kaempferol, the intake of kaempferol showed the decreased exercise intensity and the decreased rating of perceived exertion, at the both oxygen consumption (VO$_2$) levels (stair climbing and jogging).

| Scale | Perceived exertion | Scale | Perceived exertion |
|---|---|---|---|
| 20 | Maximally hard | 11 | Fairly light |
| 19 | Very very hard | 9 | Very light |
| 17 | Very hard | 7 | Very very light |
| 15 | Hard | 6 | Comfort |

(continued)

| Scale | Perceived exertion | Scale | Perceived exertion |
|---|---|---|---|
| 13 | Somewhat hard | | |

As indicated in the results of the above 1 to 6, ingesting the kaempferol-containing food increased the oxygen consumption and the oxygen consumption efficiency at the same exercise intensities. In addition, the maximum exercise load increased. In addition, comparing the exercise intensities at the same oxygen consumption, it is found that the heart rate was decreased, which suggests that the subjects could exercise more comfortably with less breathlessness due to increased endurance. This improvement in physical activity efficiency may also reduce the fatigue of the subject. In fact, the ingestion of kaempferol-containing foods reduced perceived exertion and reduced breathlessness and fatigue. Therefore, it is suggested that the composition may be used for reducing breathlessness, and/or an enhancing endurance.

<7: Effect on dynamic/kinetic visual acuity>

The effect on dynamic/kinetic visual acuity was examined by measuring dynamic visual acuity (DVA) and kinetic visual acuity (KVA) before and after (within about 1 min after exercise) the incremental loading exercise.
(1) Measurement of dynamic visual acuity (DVA) Measurement equipment:
Dynamic vision tester HI-10 Dynamic Vision Tester (Kowa Company, Ltd.)

Measurement Method:

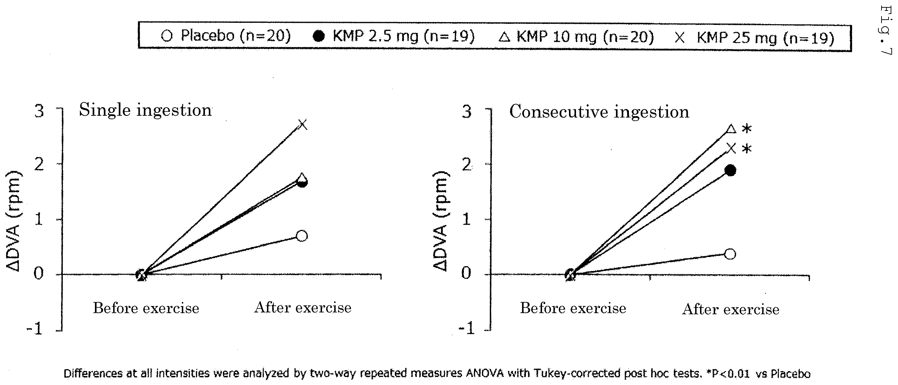

The fastest visible speed of Landolt's ring moving horizontally on the arc was measured with the subject as the center. The target automatically decelerated gradually as it rotated, and when the subject detected the break in Landolt's ring, the subject pressed a switch on his/her hand to indicate the direction (up and down, and left and right) of the break. The number of revolutions at the time of the digital display was recorded on the recording paper and used as the inspection result. The results are shown in Fig. 7.

<Test Example 2: Effect on ATP production in hypoxic environment>

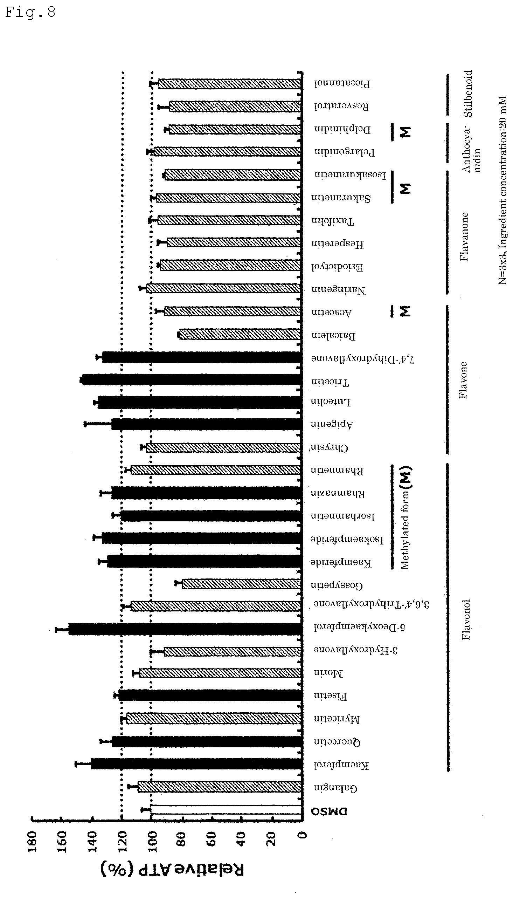

$C_2C_{12}$ skeletal muscle cells differentiated with horse serum were obtained, and various compounds (Final concentration of 20 $\mu$M) or dimethyl sulfoxide (DMSO) as a negative control were added thereto. After incubation in a hypoxic incubator (3% $O_2$) for 24 hours, the ATP content in the cells was determined using a kit (luciferase luminescence assay) manufactured by TOYO B-Net Co., Ltd. The ATP content of the negative control treated with DMSO was defined as 100%, and the activity value of the test compounds was calculated as a percentage based on the value of DMSO.
The results are shown in Fig. 8.

<Test Example 3: Effect of kaempferol or kaempferol 3-O-glucoside in rat>

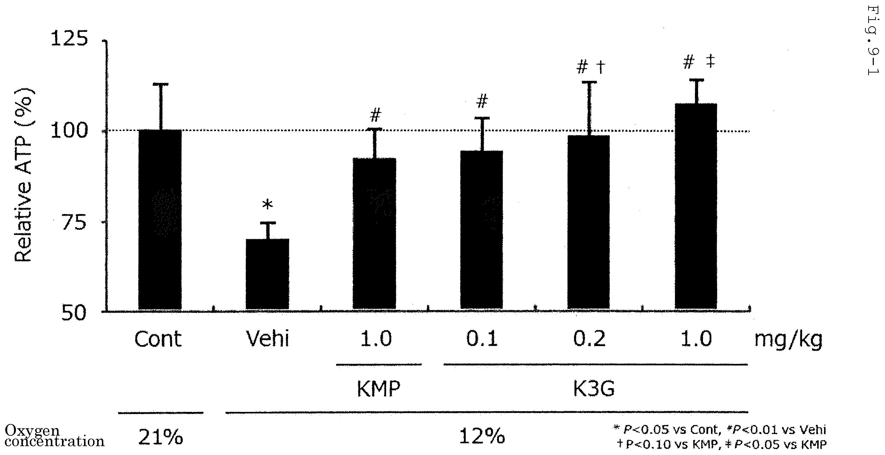

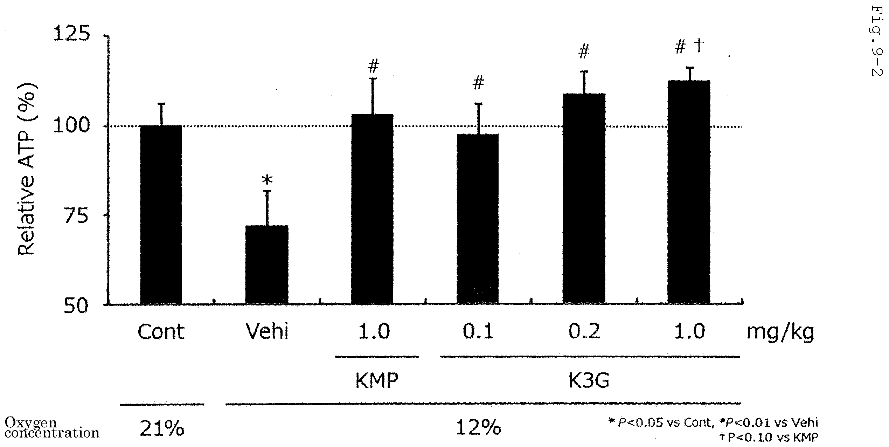

Male Sprague-Dawley rats at 9 weeks of age were orally administered kaempferol (KMP; 1.0 mg/kg body weight) or kaempferol 3-O-glucoside (K3G; 0.1, 0.2, or 1 mg/ kg body weight (KMP aglycone equivalent value)) once a day for 8 consecutive days at 9 AM (reared in 21% oxygen). On day 8 of administration, the control group was exposed to 21% oxygen for 1 hour, and the other groups were exposed to 12% oxygen for 1 hour. Then, the soleus muscle (Sol) and whole brain were excised, and the ATP contents in the tissues were measured. The results are shown in Figs. 9-1 and 9-2.

<Test Example 4: Amounts of kaempferol and kaempferol glycosides in raw materials>

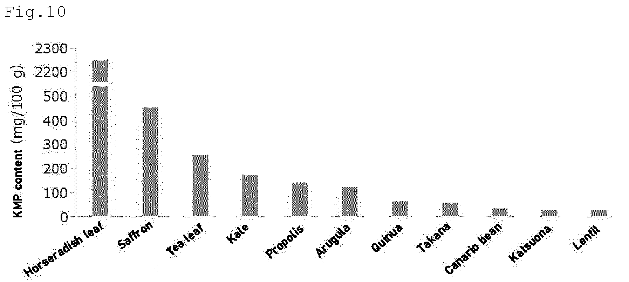

The amounts of kaempferol and kaempferol glycosides contained in horseradish leaf, saffron, black tea leaf, kale, propolis, Takana, and arugula (test samples) were measured according to the following method. The results are shown in FIG. 10.

(1) In a 50 mL PP tube, 0.8 g of a test sample was weighed with an electronic scale (N = 1 to 3). A 70 % aqueous ethanol solution (40 mL) measured with a graduated cylinder was added thereto. The mixture was homogenized with a polytron homogenizer at 2,000 rpm for 1 minute at room temperature.

(2) The suspension was thoroughly reversed and mixed, and 1 mL of the suspension was dispensed into a 14 mL glass tube with a 1 mL PIPETMAN (N = 1 to 3).

(3) 1 mL of 2N hydrochloric acid was added thereto with a 1 mL PIPETMAN, and the mixture was mixed with vortex, and heated in a heat block at 100 °C for 20 minutes (conversion of a kaempferol glycoside(s) into kaempferol aglycone by acid hydrolysis). The mixture was cooled in ice for more than 5 minutes or more after the heating.

(4) In a fume hood, 5 mL of hexane was added thereto by a dispenser. The mixture was shaken horizontally 20 times with hands. The mixture was centrifuged at 4 °C, 12,000 rpm, for 5 minutes in a large cooling centrifuge.

(5) In a fume hood, the upper layer was discarded with a Pasteur pipette. Ethyl acetate (5 mL) was added the residue by a dispenser. The mixture was shaken horizontally with a shaker at room temperature, 150 rpm, for 10 minutes. The mixture was centrifuged at 4 °C, 12,000 rpm, for 5 minutes in a large cooling centrifuge. (6) In a fume hood, the upper layer was collected with a Pasteur pipette into a 14 mL glass tube for drying, and subjected to nitrogen drying at 60 °C in a spray type nitrogen drying machine.

To the lower layer was added 5 mL of ethyl acetate by a dispenser. The mixture was shaken horizontally at room temperature, 150 rpm, for 10 minutes with a shaker. The mixture was centrifuged at 4 °C, 12,000 rpm, for 5 minutes in a large cooling centrifuge. (7) The 14 mL glass test tube for drying was took out (it is not necessary to be dried completely).

In a fume hood, the upper layer was collected with a Pasteur pipette into the 14 mL glass tube for drying, and subjected to nitrogen drying at 60 °C in a spray type nitrogen drying machine.

(8) After the drying was completed, the 14 mL glass tube was took out of the machine, 1 mL of a 70% aqueous ethanol solution was added thereto by a 1 mL PIPETMAN. The mixture was mixed by vortex. The mixture was mixed ultrasonically for 1 minute twice with an ultrasonic generator, and then was mixed by vortex to provide a solution. 400 mL of the solution was added to a Mini-Uni Filter with a 1 mL PIPETMAN and passed through the filter.

(9) HPLC analysis was conducted.

<Test Example 5: Production of horseradish extract>

A horseradish extract was produced by the following steps (1) to (6).

(1) Horseradish leaves were harvested, washed, sterilized, and dried by hot air.
(2) The dried horseradish leaves (kaempferol content (aglycone equivalent) 11 mg/g) were extracted with 50% ethanol. The resulting mixture was concentrated.
(3) The extract concentrate was adsorbed on and eluted from a resin column to purify and concentrate kaempferol.
(4) The purified liquid was adjusted to pH 5.0 with an appropriate acid or base, and subjected to an enzymatic treatment with a hydrolase at 50 °C for an appropriate time.
(5) The enzyme was inactivated at 80 °C.
(6) Lyophilization was performed to provide a horseradish extract (powder).

<Test Example 6: Quantitative analysis of kaempferol in horseradish extract>

The amount of kaempferol aglycone contained in the horseradish extract (powder, dry weight) obtained in Test Example 5 was determined by the following method. The kaempferol content in horseradish extract (powder, dry weight) was 161 mg/g.

Method

(1) In a 50 mL PP tube, 0.2 g of a test sample was weighed with an electronic scale (N = 1 to 3). A 70 % aqueous ethanol solution (40 mL) measured with a graduated cylinder was added thereto. The mixture was homogenized with a polytron homogenizer at 2,000 rpm for 1 minute at room temperature.
(2) The suspension was thoroughly reversed and mixed, and 1 mL of the suspension was dispensed into a 14 mL glass tube with a 1 mL PIPETMAN (N = 1 to 3).
(3) 1 mL of Milli-Q was added thereto with a 1 mL PIPETMAN, and the mixture was mixed with vortex, and the mixture was heated in a heat block at 100 °C for 20 minutes. The mixture was cooled in ice for 5 minutes or more after the heating.
(4) In a fume hood, 5 mL of hexane was added thereto by a dispenser. The mixture was shaken horizontally 20 times with hands. The mixture was centrifuged at 4 °C, 12,000 rpm, for 5 minutes in a large cooling centrifuge.

(5) In a fume hood, the upper layer was discarded with a Pasteur pipette. Ethyl acetate (5 mL) was added the residue by a dispenser. The mixture was shaken horizontally with a shaker at room temperature, 150 rpm, for 10 minutes. The mixture was centrifuged at 4 °C, 12,000 rpm, for 5 minutes in a large cooling centrifuge.

(6) In a fume hood, the upper layer was collected with a Pasteur pipette into a 14 mL glass tube for drying, and subjected to nitrogen drying at 60 °C in a spray type nitrogen drying machine.

To the lower layer was added 5 mL of ethyl acetate by a dispenser. The mixture was shaken horizontally at room temperature, 150 rpm, for 10 minutes with a shaker. The mixture was centrifuged at 4 °C, 12,000 rpm, for 5 minutes in a large cooling centrifuge. (7) The 14 mL glass test tube for drying was took out (it is not necessary to be dried completely).

In a fume hood, the upper layer was collected with a Pasteur pipette into the 14 mL glass tube for drying, and subjected to nitrogen drying at 60 °C in a spray type nitrogen drying machine.

(8) After the drying was completed, the 14 mL glass tube was took out of the machine, 10 mL of a 70% aqueous ethanol solution was added thereto by a 10 mL PIPETMAN. The mixture was mixed by vortex. The mixture was mixed ultrasonically for 1 minute twice with an ultrasonic generator, and then was mixed by vortex to provide a solution. 400 mL of the solution was added into a Mini-Uni Filter with a 1 mL PIPETMAN and passed through the filter.

(9) HPLC analysis was conducted.

## Claims

1. A composition for improvement in physical activity efficiency, comprising a plant-derived extract selected from a horseradish extract, a saffron extract, a tea leaf extract, a kale extract, a propolis extract, a Takana extract, an arugula extract, and a mixture thereof, and/or a plant-derived processed product selected from a horseradish processed product, a saffron processed product, a tea leaf processed product, a kale processed product, a propolis processed product, a Takana processed product, an arugula processed product, and a mixture thereof.

2. The composition according to claim 1, wherein the improvement in physical activity efficiency is improvement in endurance.

3. The composition according to claim 1, wherein the improvement in physical activity efficiency is reduction of breathlessness.

4. A composition for reducing fatigue, comprising a plant-derived extract selected from a horseradish extract, a saffron extract, a tea leaf extract, a kale extract, a propolis extract, a Takana extract, an arugula extract, and a mixture thereof, and/or a plant-derived processed product selected from a horseradish processed product, a saffron processed product, a tea leaf processed product, a kale processed product, a propolis processed product, a Takana processed product, an arugula processed product, and a mixture thereof.

5. A composition for improving dynamic/kinetic visual acuity, comprising a plant-derived extract selected from a horseradish extract, a saffron extract, a tea leaf extract, a kale extract, a propolis extract, a Takana extract, an arugula extract, and a mixture thereof, and/or a plant-derived processed product selected from a horseradish processed product, a saffron processed product, a tea leaf processed product, a kale processed product, a propolis processed product, a Takana processed product, an arugula processed product, and a mixture thereof.

6. The composition according to any one of claims 1 to 5, comprising a horseradish extract.

7. The composition according to any one of claims 1 to 6, comprising a horseradish processed product.

8. The composition according to any one of claims 1 to 7, wherein the plant-derived extract and/or the plant-derived processed product comprises kaempferol.

9. The composition according to any one of claims 1 to 8, wherein the amount of kaempferol comprised in the composition is 0.1 mg to 200 mg.

10. The composition according to any one of claims 1 to 9, wherein said composition is for administration at a dose of 0.1 mg to 200 mg of kaempferol per administration.

**11.** The composition according to any one of claims 1 to 10, wherein said composition is for administration at a dose of 0.5 mg to 100 mg of kaempferol per administration.

**12.** The composition of any one of claims 1 to 11, wherein said composition is for administration to a subject who is hypoxic.

**13.** The composition according to any one of claims 1 to 12, wherein said composition is a food and drink.

**14.** The composition according to any one of claims 1 to 12, wherein said composition is a pharmaceutical composition.

Fig. 1

| O Placebo (n=20) | ● KMP 2.5 mg (n=19) | △ KMP 10 mg (n=20) | X KMP 25 mg (n=19) |

Differences at all intensities were analyzed by two-way repeated measures ANOVA with Tukey-corrected post hoc tests.

KMP 2.5 mg: [a]$P<0.01$, [a']$P<0.001$, [a'']$P<0.0001$ vs Placebo; KMP 10 mg: [b]$P<0.0001$ vs Placebo, [c]$P<0.0001$ vs Placebo

Fig. 2

○ Placebo (n=20)   ● KMP 2.5 mg (n=19)   △ KMP 10 mg (n=20)   ✕ KMP 25 mg (n=19)

Single ingestion

Consecutive ingestion

Start of incremental
loading exercise

Start of incremental
loading exercise

Differences at all intensities were analyzed by two-way repeated measures ANOVA with Tukey-corrected post hoc tests.

KMP 2.5 mg: [a]$P<0.05$, [a']$P<0.01$, [a"]$P<0.01$ vs Placebo; KMP 10 mg: [b]$P<0.05$, [b']$P<0.01$, [b"]$P<0.001$ vs Placebo; [c]$P<0.05$, [c']$P<0.01$, [c"]$<0.001$ vs Placebo

Single ingestion

Consecutive ingestion

Differences at all intensities were analyzed by two-way repeated measures ANOVA with Tukey-corrected post hoc tests. *$P<0.05$, #$P<0.0001$ vs Placebo

EP 3 932 485 A1

Fig. 3

Fig. 4

EP 3 932 485 A1

Differences at all intensities were analyzed by two-way repeated measures ANOVA with Tukey-corrected post hoc tests. *$P<0.001$, #$P<0.0001$ vs Placebo

Fig.5

Fig.6-1

VO2 in the level of : 14 mL/kg/min climbing stairs

Single ingestion

Exercise intensity in the level of climbing stairs (%HR)

| | Placebo | 2.5 mg | 10 mg | 25 mg |
|---|---|---|---|---|
| RPE | 11.7 | 10.5* | 9.7*# | 9.7* |
| Perceived exertion | Fairly light | Fairly light | Very light | Very light |

Consecutive ingestion

Exercise intensity in the level of climbing stairs (%HR)

| | Placebo | 2.5 mg | 10 mg | 25 mg |
|---|---|---|---|---|
| RPE | 11.1 | 9.5* | 9.2*# | 9.0*# |
| Perceived exertion | Fairly light | Very light | Very light | Very light |

Differences at all intensities were analyzed by two-way repeated measures ANOVA with Tukey-corrected post hoc tests.
*P<0.01, #P<0.001, $P<0.0001 vs Placebo

Fig. 6-2

EP 3 932 485 A1

$$\frac{\text{VO}_2 \text{ in the}}{\text{level of jogging}} : 24.5 \,\text{mL/kg/min}$$

Single ingestion

Consecutive ingestion

| RPE | 17.5 | 15.9* | 15.4# | 15.4* |
|---|---|---|---|---|
| Perceived exertion | Very hard | Hard | Hard | Hard |

| RPE | 17.2 | 15.2* | 14.7# | 15.1* |
|---|---|---|---|---|
| Perceived exertion | Very hard | Hard | Hard | Hard |

Differences at all intensities were analyzed by two-way repeated measures ANOVA with Tukey-corrected post hoc tests.
*$P<0.05$, #$P<0.001$, $P<0.0001$ vs Placebo

Fig. 7

O Placebo (n=20)　● KMP 2.5 mg (n=19)　△ KMP 10 mg (n=20)　X　KMP 25 mg (n=19)

Single ingestion

Consecutive ingestion

Differences at all intensities were analyzed by two-way repeated measures ANOVA with Tukey-corrected post hoc tests. *P<0.01 vs Placebo

Fig.8

Fig.9-1

Fig.9-2

Fig.10

| | International application No. |
|---|---|
| | PCT/JP2020/007858 |

**A.  CLASSIFICATION OF SUBJECT MATTER**
A61P  3/00(2006.01)i;  A61P  3/02(2006.01)i;  A61P  11/00(2006.01)i;  A61P 27/02(2006.01)i;  A61K  36/31(2006.01)i;  A61K  36/82(2006.01)i;  A61K 36/88(2006.01)i; A61K 31/352(2006.01)i; A61K 35/644(2015.01)i
FI:     A61K36/31; A61K36/88; A61K36/82; A61K35/644; A61P27/02; A61P3/02; A61P3/00; A61P11/00; A61K31/352
According to International Patent Classification (IPC) or to both national classification and IPC

**B.  FIELDS SEARCHED**

Minimum documentation searched (classification system followed by classification symbols)
A61P3/00;  A61P3/02;  A61P11/00;  A61P27/02;  A61K36/31;  A61K36/82; A61K36/88; A61K31/352; A61K35/644

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)
JSTPlus/JMEDPlus/JST7580 (JDreamIII); Caplus/MEDLINE/EMBASE/BIOSIS/WPIDS (STN)

**C.  DOCUMENTS CONSIDERED TO BE RELEVANT**

| Category* | Citation of document, with indication, where appropriate, of the relevant passages | Relevant to claim No. |
|---|---|---|
| X | JP 2009-155333 A (TSUJIDO CHEMICAL CORP.) 16.07.2009 (2009-07-16) examples 1, 2, paragraph [0024] | 1-3, 6-14 |
| Y | HABBU, PV. et al., "Adaptogenic and in vitro antioxidant activity of flavanoids and other fractions of Argyreia speciosa (Burm. f) Boj. in acute and chronic stress paradigms in rodents.", Indian Journal of Experimental Biology, 2010, vol.48, pp. 53-60 abstract, page 54, right column, lines 7-15, page 57, left column, lines 12-20, fig. 2 | 1-3, 6-14 |
| Y | BILYK, A. et al., "Distribution of Quercetin and Kaempferol in Lettuce, Kale, Chive, Garlic, Chive, Leek, Horseradish, Red Radish, and Red Cabbage Tissues.", J. Agric. Food Chem., 1985, vol. 33, pp. 226-228 table 3 | 1-3, 6-14 |

☒   Further documents are listed in the continuation of Box C.   ☒   See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 May 2020 (08.05.2020) | 19 May 2020 (19.05.2020) |

| Name and mailing address of the ISA/ Japan Patent Office 3-4-3, Kasumigaseki, Chiyoda-ku, Tokyo 100-8915, Japan | Authorized officer |
|---|---|
| | Telephone No. |

Form PCT/ISA/210 (second sheet) (January 2015)

**INTERNATIONAL SEARCH REPORT**

International application No.

PCT/JP2020/007858

C (Continuation).   DOCUMENTS CONSIDERED TO BE RELEVANT

| Category* | Citation of document, with indication, where appropriate, of the relevant passages | Relevant to claim No. |
|---|---|---|
| P, X | WO 2019/043846 A1 (OTSUKA PHARMACEUTICAL CO., LTD.) 07.03.2019 (2019-03-07) claims, paragraph [0003], examples | 1-3, 6-14 |
| P, X | WO 2019/044964 A1 (OTSUKA PHARMACEUTICAL CO., LTD.) 07.03.2019 (2019-03-07) claims, paragraph [0003], examples | 1-3, 6-14 |
| A | CN 102138998 A (DRUG & INSTRUMENT INSPECTION INSTITUTE, MINISTER OF HEALTH, P. L.A. THE GENERAL LOGISTICS DEPARTMENT) 03.08.2011 (2011-08-03) examples 1-4, tables 1-3 | 1-3, 6-14 |

Form PCT/ISA/210 (continuation of second sheet) (January 2015)

<table>
<tr><td colspan="2" style="text-align:center"><strong>INTERNATIONAL SEARCH REPORT</strong></td><td>International application No.<br>PCT/JP2020/007858</td></tr>
</table>

**Box No. II**   **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**   **Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.: Parts with respect to "horseradish" in the invention relating to an "exercise efficiency improvement composition" described in claim 1 and claims 2, 3, and 6-14 dependent on claim 1

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.

PCT/JP2020/007858

| Patent Documents referred in the Report | Publication Date | Patent Family | Publication Date |
|---|---|---|---|
| JP 2009-155333 A | 16 Jul. 2009 | (Family: none) | |
| WO 2019/043846 A1 | 07 Mar. 2019 | TW 201912154 A | |
| WO 2019/044964 A1 | 07 Mar. 2019 | TW 201912154 A | |
| CN 102138998 A | 03 Aug. 2011 | (Family: none) | |

Form PCT/ISA/210 (patent family annex) (January 2015)

**INTERNATIONAL SEARCH REPORT**

International application No.

PCT/JP2020/007858

<Continuation of Box No. III>

Document 1: JP 2009-155333 A (TSUJIDO CHEMICAL CORP.) 16.07.2009 (2009-07-16) examples 1, 2, paragraph [0024] (Family: none)

Document 2: CN 102138998 A (DRUG & INSTRUMENT INSPECTION INSTITUTE, MINISTER OF HEALTH, P. L. A. THE GENERAL LOGISTICS DEPARTMENT) 03.08.2011 (2011-08-03) examples 1-4, tables 1-3 (Family: none)

Document 3: HABBU PV. et al., "Adaptogenic and in vitro antioxidant activity of flavanoids and other fractions of Argyreia speciosa (Burm. f) Boj. in acute and chronic stress paradigms in rodents.", Indian Journal of Experimental Biology, 2010, vol.48, pp. 53-60

(1) Invention in claims classified as "main invention"

A. Determination of the "main invention" on the basis of the special technical feature
Claim 1 sets forth the invention relating to an "exercise efficiency improvement composition" including extract or processed products of seven types of plants such as horseradish.
Meanwhile, document 1 indicates that: stem extract of Sawa-wasabi (Japanese horseradish) was dissolved in pure water and fed to a mouse orally, the mouse was put in a water bath, and thus the time taken until the mouse was immobilized was longer than a control group (example 1); the extract was fed to a volunteer orally, the volunteer was exercising on a bicycle ergometer, and thus the increase rate of the lactic acid value in the blood was lower than a placebo group (example 2); and using horseradish as a raw material also showed the same results (paragraph [0024]). Here, that the time taken until the mouse was immobilized in the water bath was prolonged or the increase rate of the urine acid value in the blood after exercising was lower corresponds to "exercise efficiency improvement" and "endurance improvement."
Thus, the invention relating to "horseradish" which is the first option in the invention relating to an "exercise efficiency improvement composition" including extract or processed products of seven types of plants set forth in claim 1 lacks novelty in light of document 1, and thus does not have a special technical feature.
Parts with respect to "horseradish" in the invention in claim 2 referring to claim 1 lack novelty in light of document 1, and thus do not have a special technical feature.

Therefore, the parts with respect to an exercise efficiency improvement composition including extract or processed products of "horseradish" in the invention in claims 1 and 2, which have been evaluated with respect to whether or not a special technical feature is present, are classified as the "main invention."

B. Determination of unity of independent claims and dependent claims
The parts with respect to an "exercise efficiency improvement composition" including extract or processed products of "horseradish" in the invention in claims 3 and 6-14 dependent on claim 1 are classified as a "main invention."

Form PCT/ISA/210 (extra sheet) (January 2015)

C. Determination of invention substantially identical or equivalent thereto

The parts with respect to six types of plants other than "horseradish" in the invention in claims 1-3 and 8-14 are not associated with any of: an invention differing from the invention classified as the "main invention" only in terms of how the invention is described, the invention being obtained by adding well-known or common features to the invention classified as the "main invention", deleting features therefrom, or converting features of the invention classified as the "main invention", and which does not exhibit a novel effect; and an invention in which the difference with the invention classified as the "main invention" is "a design modification resulting from the specific application of a technique", or "optimization or an increase in the appropriateness of a numerical range," wherein it can be easily determined that this difference does not result in a beneficial effect in comparison to cited inventions, and thus are not classified as the "main invention."

(In addition, by paragraph [0003] of the present description, it will be understood that there is a common feature in that all compositions including extract or processed products of seven types of plants set forth in claims 1 and 2 are exercise efficiency improvement (or endurance improvement) compositions derived from plants containing kaempferol.

However, document 2 indicates that extract derived from Hovenia dulcis containing 0.5-1% of kaempferol is administered by gavage to rats, and as a result, energy storage reduction after running, accumulation of metabolites, radical excess, etc. are improved, and the extract improves a survival of rat cardiomyocytes cultured in the anaerobic conditions and oxygen deficiency in rats under low oxygen are improved (corresponding to "exercise efficiency improvement" or "endurance improvement") (examples 1-4, tables 1-3), and document 3 indicates that swimming time is prolonged in mice orally administered with kaempferol derived from argyreia nervosa (corresponding to "exercise efficiency improvement" or "endurance improvement") (abstract, page 54, right column, lines 7-15, page 57, left column, lines 12-16, fig. 2).

Therefore, the exercise efficiency improvement (or endurance improvement) composition including extract or processed products of plants containing kaempferol is known before the priority date of the present application, and thus compositions including extract or processed products of seven types of plants set forth in claims 1 and 2 do not have any technical feature to make a contribution over the prior art. For this reason, these seven inventions do not comply with the requirement of unity of invention.)

Form PCT/ISA/210 (extra sheet) (January 2015)

**INTERNATIONAL SEARCH REPORT**

International application No.

PCT/JP2020/007858

In addition, claims 4 and 6-14 set forth the invention relating to a "fatigue alleviation composition" including extract or processed products of seven types of plants, and claims 5-14 set forth the invention relating to a "dynamic vision improvement composition" including extract or processed products of seven types of plants, all of which are not associated with any of: an invention differing from the invention classified as the "main invention" only in terms of how the invention is described, the invention being obtained by adding well-known or common features to the invention classified as the "main invention", deleting features therefrom, or converting features of the invention classified as the "main invention", and which does not exhibit a novel effect; and an invention in which the difference with the invention classified as the "main invention" is "a design modification resulting from the specific application of a technique", or "optimization or an increase in the appropriateness of a numerical range," wherein it can be easily determined that this difference does not result in a beneficial effect in comparison to cited inventions, and thus are not classified as the "main invention."

(2) "Number of inventions" included in the claims

The present application can be said to include a total of 21 inventions, the seven inventions relating to an "exercise efficiency improvement composition" including extract or processed products of seven types of plants set forth in claims 1-3 and 6-14, the seven inventions relating to a "fatigue alleviation composition" including extract or processed products of seven types of plants set forth in claims 4 and 6-14, and the seven inventions relating to a "dynamic vision improvement composition" including extract or processed products of seven types of plants set forth in claims 5-14.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014171333 A
- JP 2007228855 A
- JP 2013542924 A

### Non-patent literature cited in the description

- **WILLIAMS JH ; POWERS SK ; STUART MK.** Hemoglobin desaturation in highly trained athletes during heavy exercise. *Med Sci Sports Exerc,* 1986, vol. 18, 168-173
- **WILKINS, R.L. et al.** Clinical assessment in respiratory care. *Relationship between Age, PaO, and Saturation,* 2004
- **M. MONTERO ; C.D. LOBATON ; E. HERNAN-DEZ-SANMIGUEL et al.** Direct activation of the mitochondrial calcium uniporter by natural plant flavonoids. *Biochem. J.,* 2004, vol. 384, 19-24
- **DA-SILVA WS ; HARNEY JW ; KIM BW ; LI J ; BIANCO SD ; CRESCENZI A ; CHRISTOFFOLETE MA ; HUANG SA ; BIANCO AC.** The small polyphenolic molecule kaempferol increases cellular energy expenditure and thyroid hormone activation. *Diabetes,* 2007, vol. 56, 767-776
- Oxygen uptake effciency slope (OUES): Sono seirigaku-teki kiso to rinshō ōyō" [Oxygen uptake effciency slope (OUES): its physiological basis and clinical application. The Tokai journal of sports medical science. Tokai University, vol. 11, 9-14, 1999-03
- *Mini Rev Med Chem.,* 2011, vol. 11 (4), 298-344
- *Mini Rev Med Chem,* 2011, vol. 11 (4), 298-344